Europäisches Patentamt

European Patent Office

Office européen des brevets

(11) Numéro de publication: **0 256 903**
A1

# (12) DEMANDE DE BREVET EUROPEEN

(21) Numéro de dépôt: 87401627.2

(22) Date de dépôt: 09.07.87

(51) Int. Cl.⁴: **C 23 G 5/028**
C 07 C 17/42

(30) Priorité: 21.07.86 FR 8610528

(43) Date de publication de la demande:
24.02.88 Bulletin 88/08

(84) Etats contractants désignés:
AT BE CH DE FR GB GR IT LI LU NL SE

(71) Demandeur: ATOCHEM
4 & 8, Cours Michelet La Défense 10
F-92800 Puteaux (FR)

(72) Inventeur: Campos, Michel
36 Rue de la Marne
F-78800 Houilles (FR)

Boussaguet, Jean-Charles
11 Rue Alphonse Daudet
F-78410 Aubergenville (FR)

Letullier, Jean-Philippe
15 Rue des Coutures
F-92190 Meudon (FR)

(54) Composition à base de chlorure de méthylène - son utilisation pour le dégraissage des métaux.

(57) L'invention a pour objet une composition à base de chlorure de méthylène et son utilisation pour le dégraissage des métaux, en particulier les alliages légers. On ajoute au chlorure de méthylène de l'oxyde de propylène, de l'acétone et du méthyl-tert-butyléther.

EP 0 256 903 A1

## Description

COMPOSITION A BASE DE CHLORURE DE METHYLENE SON UTILISATION POUR LE DEGRAISSAGE DES METAUX

La présente invention concerne une composition à base de chlorure de méthylène et son utilisation pour dégraisser les métaux.

Les hydrocarbures chlorés grâce à leur pouvoir solvant, leur ininflammabilité, leur point d'ébullition relativement bas sont le plus souvent employés pour le dégraissage des métaux, des textiles, des céramiques et des verres.

Le chlorure de méthylène est particulièrement stable à l'oxydation, l'hydrolyse, la pyrolyse. Il ne participe à aucune réaction photochimique. Son point d'ébullition bas permet son utilisation à faible température. Toutes ces propriétés en font donc un solvant de choix dans le dégraissage des métaux.

Cependant, lors de cette opération, il peut réagir avec des composés aromatiques introduits lors de l'usinage de métaux légers.

Selon un mécanisme réactionnel (type Friedel et Crafts) on a alors formation d'acide chlorhydrique, de composés plus lourds qui polluent le chlorure de méthylène. Il y a donc un risque d'explosion par décomposition (formation de chlorure d'acide, phosgène, gaz chlorhydrique).

Pour pallier ces inconvénients, il est nécessaire d'ajouter au chlorure de méthylène de petites quantités de produits organiques qui empêchent cette réaction : c'est la stabilisation.

On a déjà proposé des compositions de chlorure de méthylène stabilisé. Le brevet US 3,887,628 décrit du chlorure de méthylène contenant 0,05 à 2 % d'oxyde de butylène et d'oxyde de propylène. Le brevet US 3,923,912 décrit du chlorure de méthylène contenant 0,05 à 2 % d'acétone ou de méthyléthylcétone et éventuellement de l'oxyde de propylène. Le brevet US 4,108,910 décrit du chlorure de méthylène contenant un époxyde et un monoéther aliphatique. Le brevet européen EP 11 658 décrit du chlorure de méthylène stabilisé par du formiate d'éthyle ou de méthyle.

Un but de l'invention est donc de proposer une composition utile pour le dégraissage des métaux, et qui est stable en présence de métaux légers tels que l'aluminium ou leurs alliages. Un autre but de l'invention est de fournir une composition qui puisse être régénérée facilement par distillation, c'est-à-dire qui conserve ses additifs au fur et à mesure des distillations successives.

La composition selon l'invention est du chlorure de méthylène contenant :

a/ au moins un époxyde

b/ au moins une cétone

c/ au moins un produit choisi parmi les éthers et les esters.

La quantité totale d'époxyde a/ à utiliser peut être comprise entre 0,05 et 5 % en poids de la masse totale de la composition et de préférence entre 0,2 et 2 %. Les époxydes peuvent être l'oxyde de propylène ou l'oxyde de butylène. On préfère l'oxyde de propylène.

La quantité totale de cétone b/ peut être comprise entre 0,05 et 5 % en poids de la masse totale de la composition et de préférence entre 0,2 et 2 %. Comme cétones, on utilise le plus souvent des cétones aliphatiques ayant de 3 à 10 atomes de carbone. L'acétone convient parfaitement.

La quantité totale de c/ peut être comprise entre 0,05 et 5 % en poids de la masse totale de la composition et de préférence entre 0,2 et 2 %. Comme éthers, on utilise le plus souvent des mono di et tri éthers aliphatiques ayant de 2 à 12 atomes de carbone, et des ethers cycliques n'ayant pas plus de 3 atomes de carbone dans le cycle par fonction éther. Le méthyl-tert-butyléther (MTBE) et le diméthoxyméthane conviennent particulièrement. Comme esters on utilise le plus souvent des produits de formule $R_1$

$R_2$ dans laquelle $R_1$ et $R_2$ sont des groupes alkyles et ayant chacun de 1 à 4 atomes de carbone. On préfère utiliser le formiate de méthyle. On peut utiliser un éther ou un ester ou un mélange comprenant plusieurs produits appartenant à la même famille ou aux deux familles précitées. On utilise avantageusement le MTBE seul.

La composition selon l'invention est préparée par simple mélange des produits avec le chlorure de méthylène. Ce mélange peut se faire à température ambiante et plus spécialement comprise entre 5 et 30°C.

La présente invention concerne aussi un procédé de dégraissage de métaux à l'aide de la composition selon l'invention. Le dégraissage peut se faire en phase vapeur ou en phase liquide selon des modes opératoires conventionnels.

Les exemples suivants illustrent l'invention sans la limiter.

Les concentrations sont exprimées en pourcentages pondéraux.

EXEMPLES 1 à 3

On prépare 3 compositions de formules suivantes :

0 256 903

|  | Composition 1 | Composition 2 | Composition 3 |
|---|---|---|---|
| Chlorure de méthylène | 98 | 98 | 98 |
| Oxyde de propylène | 0,5 | 0,5 | 0,5 |
| Acétone | 1 | 1 | 1 |
| MTBE | 0,5 | 0 | 0 |
| Diméthoxyméthane | 0 | 0,5 | 0 |
| Formiate de méthyle | 0 | 0 | 0,5 |

Ces compositions sont soumises à un test de stabilité en présence d'aluminium et de chlorure d'aluminium. Ce test très voisin du test BAM (Bundesanstalt für material prüfung, République Fédérale Allemande) comprend les étapes suivantes :

1) A 25 ml de composition on additionne 25 ml de toluène, 0,175 g de chlorure d'aluminium (Al Cl$_3$), 4,5 g de paillettes d'Aluminium. Le mélange est maintenu à reflux pendant 18 h dans un bain marie à 60°C.

2) Au mélange 1) on ajoute 2,5 g de stéarate de Zn puis on porte à reflux pendant 18 h.

3) On mélange 1) on ajoute 2,5 ml d'acide oleique puis on porte à reflux pendant 18 h.

4) On distille 75 ml de composition en 3 fractions de 25 ml puis on fait le test 1) sur chacune des 3 fractions.

La composition satisfait au test si elle ne présente aucune réaction au cours de ces opérations.

Les compositions précèdentes ont réussi le test.

## Revendications

1. Composition à base de chlorure de méthylène, utile pour le dégraissage des métaux, caractérisée en ce qu'elle contient

a/ au moins un époxyde

b/ au moins une cétone

c/ au moins un produit choisi parmi les éthers et les esters.

2. Composition selon la revendication 1 caractérisée en ce que l'époxyde est choisi parmi l'oxyde de propylène et l'oxyde de butylène.

3. Composition selon la revendication 1 ou 2 caractérisée en ce que la cétone est choisie parmi les cétones aliphatiques ayant de 3 à 10 atomes de carbone et est de préférence l'acétone.

4. Composition selon l'une des revendications 1 à 3 caractérisée en ce que les éthers sont des éthers aliphatiques ayant de 2 à 12 atomes de carbone et des éthers cycliques n'ayant pas plus de 3 atomes de carbone dans le cycle par fonction éther.

5. Composition selon l'une des revendications 1 à 4 caractérisée en ce que l'éther est de préférence du méthyl-tert-butyléther ou du dimethoxyméthane.

6. Composition selon l'une des revendications 1 à 5 caractérisée en ce que l'ester est un produit de formule R$_1$

R$_2$ dans laquelle R$_1$ et R$_2$ sont des groupes alkyles ayant chacun de 1 à 4 atomes de carbone et de préférence du formiate de méthyle.

7. Composition selon l'une des revendications 1 à 6 caractérisée en ce que la quantité totale de a/ est comprise entre 0,05 et 5 % en poids de la masse totale de la composition et de préférence entre 0,2 et 2 %.

8. Composition selon l'une des revendications 1 à 7 caractérisée en ce que la quantité totale de b/ est comprise entre 0,05 et 5 % en poids de la masse totale de la composition et de préférence entre 0,2 et 2 %.

9. Composition selon l'une des revendications 1 à 8 caractérisée en ce que la quantité totale de c/ est comprise entre 0,05 et 5 % en poids de la masse totale de la composition et de préférence entre 0,2 et 2 %.

10. Composition selon l'une des revendications 1 à 9 caractérisée en ce que a/ est de l'oxyde de propylène, b/ de l'acétone et c/ du méthyl-tert-butyléther.

11. Procédé de dégraissage de métaux caractérisé en ce que lesdits métaux sont mis en contact avec

3

une composition selon l'une des revendications 1 à 10.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 219 248 (DIAMOND SHAMROCK CORP.) <br> * Revendications 1,3,5,9; page 8, exemple 2 * | 1-3,6-9,11 | C 23 G    5/028 <br> C 07 C   17/42 |
| Y | * Page 8, exemple 2 * | 4,5,10 | |
| D,Y | FR-A-2 350 319 (SOLVAY & CIE) <br> * Revendications 1,2; page 2, lignes 10-21 * & US-A-4 108 910 | 4,5,10 | |
| D,A | EP-A-0 011 658 (DOW CHEMICAL) <br> * Revendications 1,2,6 * | 6 | |
| A | FR-A-2 023 061 (DOW CHEMICAL) <br> * Revendications 1-3; page 1, lignes 35,36 * | 5 | DOMAINES TECHNIQUES RECHERCHES (Int. Cl.4) <br><br> C 23 G    5/00 <br> C 07 C   17/00 |

Le présent rapport de recherche a été établi pour toutes les revendications

| Lieu de la recherche | Date d'achèvement de la recherche | Examinateur |
|---|---|---|
| LA HAYE | 09-11-1987 | TORFS F.M.G. |